# EUROPEAN PATENT APPLICATION

(11) **EP 4 104 759 A1**
(43) Date of publication of application: **21.12.2022**
(21) Application number: 22178411.9
(22) Date of filing: 10.06.2022
(51) Int. Cl.: A61B 5/11, A61B 5/113, A61B 5/18, A61B 5/00, B60K 28/06

(54) **SYSTEM FOR SENSING AN OPERATOR'S PSYCHOPHYSIOLOGICAL STATE**

(30) Priority: 18.06.2021 US 202117351417
(71) Applicant: Honeywell International Inc., Charlotte, NC 28202 (US)
(72) Inventor: ATASSI, Hicham, Charlotte, 28202 (US); AVERKOVA, Dariia, Charlotte, 28202 (US); CIGANEK, Jan, Charlotte, 28202 (US); BLAHA, Bohdan, Charlotte, 28202 (US); KOLCAREK, Pavel, Charlotte, 28202 (US)
(74) Representative: LKGlobal UK Ltd.

(57) **Abstract**

Methods and systems are provided for measuring the capacitive state of an operator. The method includes placing an matrix array of pressure detection sensors in physical contact with the individual. The matrix array is integrated into the seat of the operator and continually measures the operator's presence and movement in the seat based on data received from the matrix array of pressure detection sensors. The breathing, movement and actigraphy patterns of the operator are measured and calculated based on the data received from the matrix array of pressure detection sensors. The breathing, movement and actigraphy patterns of the operator are continually analyzed to determine the capacitive state of the operator.

## Description

### TECHNICAL FIELD

The present invention generally relates to biological parameters monitoring, and more particularly relates to a system and method for determining the psychophysiological state of an individual.

### BACKGROUND

Maintaining an alert psychophysiological state is obviously important for many jobs including aircraft pilots, vehicle drivers, etc. Various types of sensors and monitors exist to monitor individuals in these positions. However, these systems are often based on recording and analysis of a single type of bio-signal and hence are prone to partial or complete loss of function. Hence, there is a need for a system and method that measures the capacitive state of an individual based on several types of bio-signals and behavior that may be derived from readouts of a pressure sensor.

### BRIEF SUMMARY

This summary is provided to describe select concepts in a simplified form that are further described in the Detailed Description. This summary is not intended to identify key or essential features of the claimed subject matter, nor is it intended to be used as an aid in determining the scope of the claimed subject matter.

A method is provided for determining the capacitive state of an operator. The method comprises: placing an matrix or array of pressure detection sensors in physical contact with the individual, where the matrix array is integrated into a seat of the operator; continually measuring the operator's presence and movement in the seat based on data received from the matrix array of pressure detection sensors; calculating breathing patterns of the operator based on the data received from the matrix array of pressure detection sensors; measuring movement patterns of the operator based on the data received from the matrix array of pressure detection sensors; calculating actigraphy patterns of the operator based on the data received from the matrix of pressure detection sensors and continually analyzing the breathing, movement and actigraphy patterns of the operator to determine the psychophysiological state of the operator.

A system is provided for detecting the capacitive state of an operator. The system comprises: an matrix of pressure detection sensors in physical contact with the operator, where the matrix is integrated into a seat of the operator; and a microprocessor that receives data from the matrix of pressure detection sensors measuring the operator's presence and movement in the seat, where the microprocessor, calculates breathing patterns of the operator based on the data received from the matrix array of pressure detection sensors; calculates movement patterns of the operator based on the data received from the matrix of pressure detection sensors; calculates actigraphy patterns of the operator based on the data received from the matrix of pressure detection sensors and continually analyzes the breathing, movement and actigraphy patterns of the operator to determine the psychophysiological state of the operator.

Furthermore, other desirable features and characteristics of the method and system will become apparent from the subsequent detailed description and the appended claims, taken in conjunction with the accompanying drawings and the preceding background.

### BRIEF DESCRIPTION OF THE DRAWINGS

The present invention will hereinafter be described in conjunction with the following drawing figures, wherein like numerals denote like elements, and wherein:
FIG. 1 shows a diagram of pressure detection sensors in physical contact with the subject individual in accordance with one embodiment;
FIG.2 shows a signal map of a pressure detection matrix array in accordance with one embodiment;
FIG. 3 shows a signal map of a pressure detection matrix array in accordance with one embodiment;
FIG.4 shows a block diagram of a pressure detection sensor and its possible integration with a dynamic multi-modal/ context-aware operator state monitoring and incapacitation detection system in accordance with one embodiment; and
FIG. 5 shows a flowchart of a method of determining the psychophysiological state of an individual using pressure detection sensors in physical contact with the operator in accordance with one embodiment.

### DETAILED DESCRIPTION

The following detailed description is merely exemplary in nature and is not intended to limit the invention or the application and uses of the invention. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary embodiments provided to enable persons skilled in the art to make or use the invention and not to limit the scope of the invention which is defined by the claims. Furthermore, there is no intention to be bound by any expressed or implied theory presented in the preceding technical field, background, brief summary, or the following detailed description.

A method and system for measuring the capacitive state of an operator has been developed. The method includes placing a matrix array of pressure detection sensors in physical contact with the individual. The matrix array is integrated into or placed on the seat of the operator and continually measures the operator's presence and movement in the seat based on data received from the matrix array of pressure detection sensors. The breathing, movement and actigraphy patterns of the operator are calculated based on the data received from the matrix array of pressure detection sensors. The breathing, movement and actigraphy patterns of the operator are continually analyzed to determine the capacitive state of the operator.

Human operators, especially in safety critical industries, such as, but not limited to transport, air transport, air traffic management, armed forces, power plants, factories, underwater operations, etc. are often required to work on rotating schedules including the shifts that are not aligned with their need for rest and thus may exceed their physiological limits. Even if the operating personnel are screened for common health conditions and risks, unforeseen and sudden health-related accidents at workplace are not uncommon, especially in the environments that put more stress on the human body.

Due to this, human operators might suffer physiological and psychophysiological decrements that would deteriorate their performance and ability to carry out their job responsibilities while on duty. Examples of such states might be, but not limited to, inattention, fatigue, drowsiness, sleep, loss of consciousness, cardiovascular and cerebrovascular events, gastro-intestinal, respiratory and otorhinolaryngological acute conditions, acute neurological and mental-health-related conditions as well as other similar debilitating ailments.

Present embodiments are a critical enabler for reduced crew and single pilot in aircraft operations. The embodiments introduce a generic approach designed for pilots. Unlike other operator monitoring systems, the present embodiment is tailored to pilot operating procedures. For example, pilots fly an aircraft and monitor all aircraft displays which is a different and more complex task than driving a car. The embodiments are scalable from a minimum viable offering using one sensor and specific function up to a complex system with multiple sensors arranged in a matrix array that provide operational capability for operations where higher confidence in the detected state of the pilot might be required.

The system uses a pressure sensor that that can be integrated with a scalable multi-modal system for a human operator state monitoring and incapacitation detection that is context aware. It takes in significant environmental conditions and it is adapted to a specific domain and the type of operations. It can be "multi-modal" in that it uses inputs from several modalities derived from traits such as visual, auditory, behavioral, postural, haptic, physiological, pupillometric, and similar performance-based traits.

A multi-modal pressure sensor proposed here includes at least one pressure detection cell sensor that is placed in/on the seat and/or backrest of the operator. It provides the information on the operator's presence in the seat and movements. A computer software algorithm transforms sensed pressure data and allows detection and monitoring of operator's movement pattern, breathing and actigraphy (rest/activity cycles).

Breathing is an informative cue that can be used for detection of a variety of states and conditions such as hypoxia, respiratory arrest, death, etc. An operator's movements and posture provide important information about the what the operator is doing and any abnormalities in the activity. Inactivity may signal extreme fatigue/, sleep, loss of consciousness, death, etc. In other circumstances, certain movement pattern may signal an epileptic seizure, inattention, distracted conditions, etc. By combining the breath, posture and actigraphy inputs from the pressure sensors it is possible to make an accurate determination of the operator's state. In other embodiments, an alert or alarm could be activated in an incapacitated state of the operator is detected.

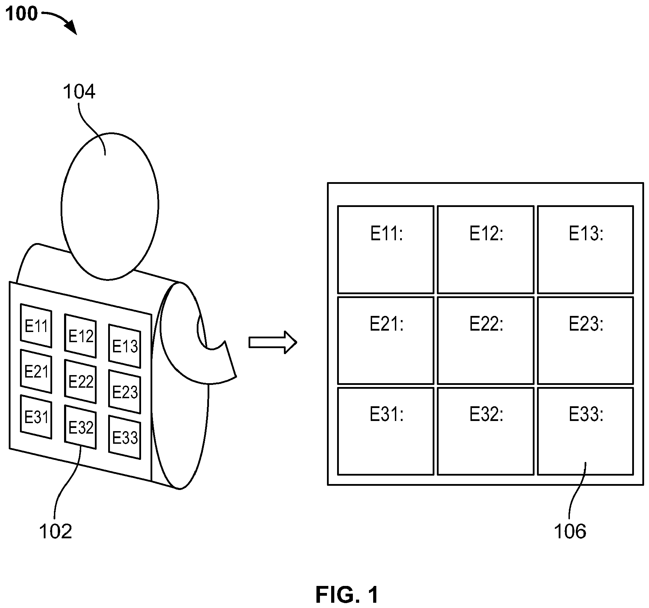

Turning now to FIG. 1, a diagram 100 is shown of a matrix array of pressure detection sensors in contact with a subject individual 104 in accordance with one embodiment. In this embodiment, the matrix array is place underneath the subject and combines two pressure detection sensors. In this example, a 3 x 3 square grid of pressure detection sensors 102 is shown. In alternative embodiments, the matrix array of pressure detection sensors could be arranged in a rectangular or similar shaped grid pattern of sensors. Additionally, the grid pattern could be longer in the horizontal and/or vertical direction with a greater number of sensors in each horizontal row and/or column. This embodiment would have the advantage of providing more sensors in contact with the back area of the subject individual 104.

The matrix array collectively measures EC voltage as a "signal" from each sensor recorded on the individual. In some embodiments, the contact with the skin of the individual is indirect because a pressure detection sensor may function with up to 1 centimeter (cm) of insulation between the individual and the sensor. This will account for any padding on the seat or clothing worn by the subject individual. In alternative embodiments, the sensors may be placed in direct physical contact with the individual. In still other embodiments, the matrix array may use resistive sensors which work on the basis of measuring changes in resistance based on the pressure applied to the sensor.

A signal is determined by the hardware of each pressure detection sensor 106. In the embodiment shown, the sensor in the lower right-hand corner of the grid (E 33) is shown to have a relative signal strength of 10. This is the highest signal in the grid sensors and it reflects the strongest signal of the matrix array. The strength of the signal is proportional to the distance between the individual and the sensor and to pressure applied on this sensor by the subject individual. Both distance and pressure are calculated for each sensor of the matrix array.

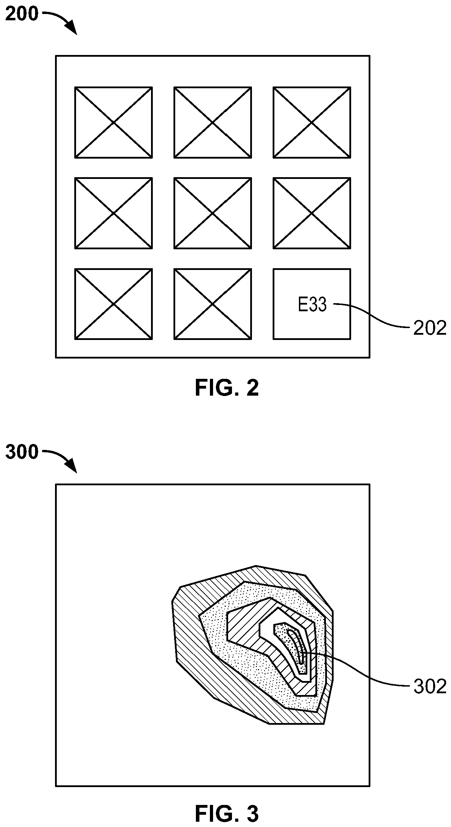

Turning now to FIG. 2, a signal map 200 is shown of a pressure detection matrix array in accordance with one embodiment. In this embodiment, the signal would merely identify E 33 sensor 202 as having the highest signal in the matrix array. The signal from E 33 would be used to provide the most reliable pressure detection voltage of all sensors of the matrix array while the signals from the other sensors in the matrix array would be ignored. In an alternative embodiment, FIG. 3 shows a signal map 300 of a pressure detection matrix array. In this embodiment, the signal s 302 of each sensor are measured and reflected on the signal map with respect to quality of the contact. In this embodiment, color coding is used to indicate the relative value of the signal strength. In this manner, the signal map can provide a more complete mapping of the distance and pressure being applied on the sensor matrix array. The map may then be used to determine the extent of breathing, movement and actigraphy by the subject individual as an independent quantity together with the pressure detection voltage.

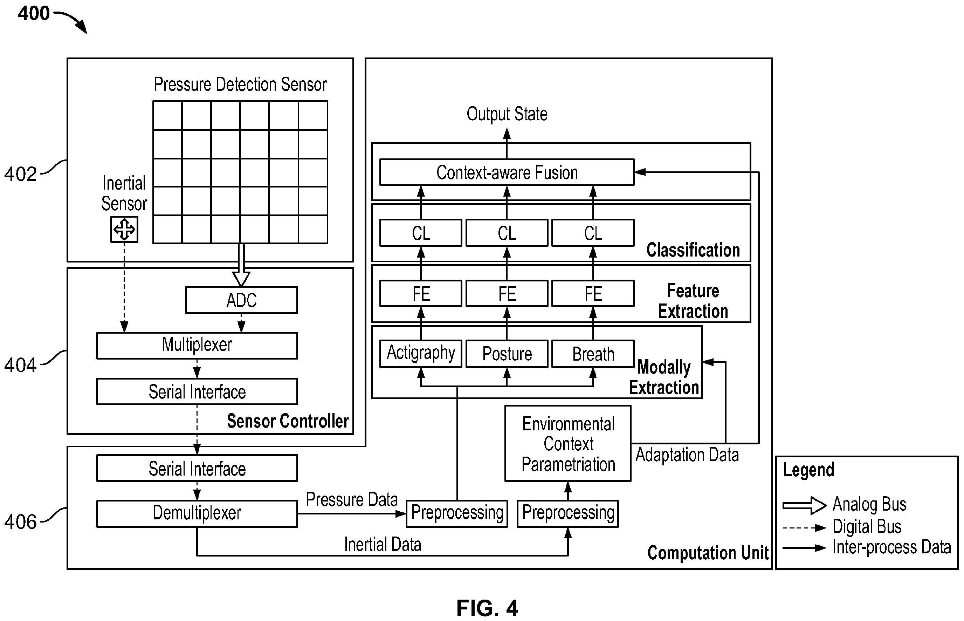

Turning now to FIG.4, a block diagram 400 is shown of a pressure detection sensor and its possible integration with a dynamic multi-modal/ context-aware operator state monitoring and incapacitation detection system in accordance with one embodiment. In this embodiment, the pressure detection sensor matrix array 402 is shown with a 5 x 6 matrix array of individual sensors along with a separate inertial sensor. Other embodiments could use different numbers of pressure and inertial sensors in different arrangements. The sensor matrix array 402 provides signal data to the sensor controller 404 which processes the data through an analog to digital converter (ADC), a multiplexer, and then a serial interface.

The serialized data in then provided to a computation unit 406 where it is received by a serial interface and de-multiplexed. The data is then split into a pressure data stream and an inertial data stream and sent for pre-processing within the computation unit 406. The pressure data is split into three categories of breathing, posture and actigraphy. The inertial data is adapted to compensate for environmental context parametrization. The pressure data and the adapted inertial data are next processed to extract the modalities and features of the specific data and then classified to determine the contextual awareness and capacity of the operator. This information may then be output in the form of an alert or alarm if the capacitive state of the operator is determined to be degraded.

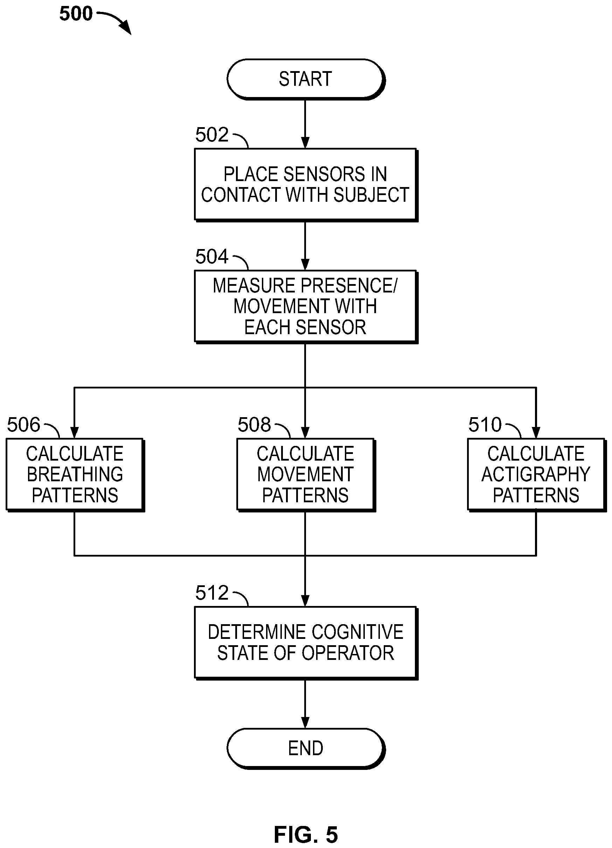

Turning now to FIG. 5, a flowchart 500 is shown of a method of determining the psychophysiological state of an individual using pressure detection sensors in physical contact with the operator in accordance with one embodiment. The method includes placing an matrix array of pressure detection sensors in physical contact with the individual 502. The matrix array is integrated into the seat of the operator and continually measures the operator's presence and movement in the seat based on data received from the matrix array of pressure detection sensors 504. The breathing 506, movement 508 and actigraphy 510 patterns of the operator are calculated based on the data received from the matrix array of pressure detection sensors. The breathing, movement and actigraphy patterns of the operator are continually analyzed to determine the capacitive state of the operator 512.

Those of skill in the art will appreciate that the various illustrative logical blocks, modules, circuits, and algorithm steps described in connection with the embodiments disclosed herein may be implemented as electronic hardware, computer software, or combinations of both. Some of the embodiments and implementations are described above in terms of functional and/or logical block components (or modules) and various processing steps. However, it should be appreciated that such block components (or modules) may be realized by any number of hardware, software, and/or firmware components configured to perform the specified functions. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention. For example, an embodiment of a system or a component may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments described herein are merely exemplary implementations.

The various illustrative logical blocks, modules, and circuits described in connection with the embodiments disclosed herein may be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor. The processor and the storage medium may reside in an ASIC. The ASIC may reside in a user terminal. In the alternative, the processor and the storage medium may reside as discrete components in a user terminal.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order without departing from the scope of the invention as long as such an interchange does not contradict the claim language and is not logically nonsensical.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth in the appended claims.

## Claims

1. A method for detecting the capacitive state of an operator, comprising:
placing a matrix array of pressure detection sensors in physical contact with the individual, where the matrix array is located on a seat of the operator;
continually measuring the operator's presence and movement in the seat based on data received from the matrix array of pressure detection sensors;
calculating breathing patterns of the operator based on the data received from the matrix array of pressure detection sensors;
measuring movement patterns of the operator based on the data received from the matrix array of pressure detection sensors;
calculating actigraphy patterns of the operator based on the data received from the matrix array of pressure detection sensors and
continually analyzing the breathing, movement and actigraphy patterns of the operator to determine the psychophysiological state of the operator.

2. The method of Claim 1, where the matrix array of pressure detection sensors comprises a rectangular shaped grid pattern of sensors.

3. The method of Claim 1, where the capacitive state of the operator is analyzed to detect an inactive state of the operator.

4. The method of Claim 3, where the inactive state of the operator is analyzed to detect excessive fatigue.

5. The method of Claim 3, where the inactive state of the operator is analyzed to detect sleep.

6. The method of Claim 3, where the inactive state of the operator is analyzed to detect hypoxia.

7. The method of Claim 3, where the inactive state of the operator is analyzed to detect respiratory arrest.

8. The method of Claim 1, where the capacitive state of the operator is analyzed to detect a seizure by the operator.

9. A system for detecting the capacitive state of an operator, comprising:
a matrix array of pressure detection sensors in physical contact with the operator, where the matrix array is integrated into a seat of the operator; and
a microprocessor that receives data from the matrix array of pressure detection sensors measuring the operator's presence and movement in the seat, where the microprocessor,
calculates breathing patterns of the operator based on the data received from the matrix array of pressure detection sensors;
measures movement patterns of the operator based on the data received from the matrix array of pressure detection sensors;
calculates actigraphy patterns of the operator based on the data received from the matrix array of pressure detection sensors and
continually analyzes the breathing, movement and actigraphy patterns of the operator to determine the psychophysiological state of the operator.

10. The system of Claim 9, where the matrix array of pressure detection sensors comprises a rectangular shaped grid pattern of sensors.

11. The system of Claim 9, where the capacitive state of the operator is analyzed to detect an inactive state of the operator.

12. The system of Claim 11, where the inactive state of the operator is analyzed to detect excessive fatigue.

13. The system of Claim 11, where the inactive state of the operator is analyzed to detect sleep.

14. The system of Claim 11, where the inactive state of the operator is analyzed to detect hypoxia.

15. The system of Claim 11, where the inactive state of the operator is analyzed to detect respiratory arrest.
